# FASCICULE DE BREVET EUROPEEN

(11) **EP 2 938 741 B1**
(45) Date de publication et mention de la délivrance du brevet: **14.02.2018**
(21) Numéro de dépôt: 13824628.5
(22) Date de dépôt: 24.12.2013
(51) Int. Cl.: C12Q 1/04, C12Q 1/10, C12Q 1/44

(54) **MILIEU DE DÉTECTION DE MICRO-ORGANISMES COMPRENANT AU MOINS UN ALKYL(THIO)GLYCOSIDE**
MIKROORGANISMUSNACHWEISMEDIUM MIT MINDESTENS EINEM ALKYL(THIO)GLYKOSID
MICRO-ORGANISM DETECTION MEDIUM COMPRISING AT LEAST ONE ALKYL(THIO)GLYCOSIDE

(30) Priorité: 28.12.2012 FR 1262965
(43) Date de publication de la demande: 04.11.2015
(73) Titulaire: bioMérieux, 69280 Marcy-l'Étoile (FR)
(72) Inventeur: CELLIER, Marie, F-38390 Montalieu-Vercieu (FR); ORENGA, Sylvain, F-01160 Neuville-sur-Ain (FR); PERRY, John, Newcastle-upon-Tyne Tyne and Wear NE7 7BH (GB)
(74) Mandataire: Murgitroyd & Company
(86) Numéro de dépôt international: PCT/FR2013/053259
(87) Numéro de publication internationale: WO 2014/102503

(56) Documents cités:
- EP-A1- 2 107 119
- WO-A1-2012/161992
- WO-A1-2014/043616
- BAUMSTUMMLER A ET AL: "Development of a nondestructive fluorescence-based enzymatic staining of microcolonies for enumerating bacterial contamination in filterable products", JOURNAL OF APPLIED MICROBIOLOGY, vol. 110, no. 1, janvier 2011 (2011-01), pages 69-79, XP002696841, ISSN: 1364-5072

## Description

### Domaine technique

La présente invention concerne l'analyse microbiologique par voie biochimique, et plus particulièrement par voie enzymatique. De manière plus spécifique, la présente invention concerne la détection de micro-organismes (par exemple de souches bactériennes) par ensemencement de milieux réactionnels, notamment aux fins de caractérisation (identification desdits micro-organismes, détermination des propriétés de résistance potentielle de ces derniers à au moins un agent antimicrobien, etc.) et/ou de dénombrement desdits micro-organismes. Ces milieux réactionnels comprennent des substrats chromogènes et/ou fluorogènes susceptibles de réagir avec les enzymes microbiennes spécifiques des micro-organismes recherchés.

### Etat de la technique

Dans le cadre de la présente invention, l'on s'intéresse plus particulièrement à la détection de micro-organismes pathogènes ou indicateurs de qualité (notamment aux fins de leur caractérisation et/ou dénombrement), que ce soit dans le milieu médical ou le milieu industriel, et plus particulièrement de micro-organismes à activité enzymatique de type estérase (comprenant notamment les activités carboxylestérase, lipase et phospholipase), osidase, peptidase, sulfatase ou phosphatase, par exemple les bactéries ou levures des genres *Salmonella, Escherichia, Pseudomonas, Listeria, Staphylococcus, Enterococcus, Candida,* et, plus spécifiquement, à la détection de bactéries du genre *Salmonella* en se basant sur la mise en évidence/détection d'une activité enzymatique estérase.

Les souches d'*Escherichia coli* sont souvent mises en évidence par la révélation d'une activité enzymatique du type osidase telle que l'activité β-glucuronidase ou β-galactosidase.

De la même façon, le genre *Listeria* est détecté par la mise en évidence de l'activité β-glucosidase.

Une activité aminopeptidase peut également être utilisée pour révéler un groupe, un genre ou une espèce de micro-organismes. L'activité L-alanine-aminopeptidase, par exemple, permet de différencier les bactéries à Gram négatif des bactéries à Gram positif.

Le genre *Salmonella,* responsable chez l'homme de diverses infections sévères (fièvre typhoïde, intoxication alimentaire), possède des estérases non-spécifiques capables d'hydrolyser des substrats synthétiques chromogènes, par exemple indigogéniques.

La détection et la caractérisation de salmonelles - et plus généralement de bactéries à activité estérase - sont classiquement réalisées en bouillon de culture ou sur des milieux gélosés qui permettent la détection et la caractérisation des colonies suspectes de bactéries à activité estérase, notamment des salmonelles. L'ensemencement de tels milieux s'opère par mise en contact de l'échantillon biologique avec le milieu.

Les bactéries à activités estérases, osidases, peptidases, sulfatases ou encore phosphatases possèdent dans leur patrimoine enzymatique des estérases, osidases, peptidases, sulfatases ou phosphatases qui clivent les liaisons cibles des substrats enzymatiques synthétiques présents dans le milieu et libèrent ainsi la partie chromophore ou fluorophore activée desdits substrats. Il en résulte une coloration ou une fluorescence qui révèle l'hydrolyse, et donc la présence de bactéries ou de colonies de bactéries cibles.

Pour pouvoir réaliser des tests de routine à grande envergure, il est nécessaire que les milieux de détection et/ou de caractérisation et/ou de dénombrement soient stables et permettent de simplifier au maximum les procédés de détection et/ou de caractérisation et/ou de dénombrement correspondants, en limitant les manipulations. En outre, il importe que les procédés offrent une très bonne sensibilité (intensité de coloration ou de fluorescence), ainsi qu'une spécificité de détection de premier ordre (afin de limiter voire éviter la détection de « faux-positifs »). La vitesse de révélation des colonies suspectes est également un paramètre fondamental de ces types de milieux et procédés de détection de bactéries présentant les activités enzymatiques susmentionnées.

Or, il est connu que les substrats synthétiques d'enzymes telles que les estérases, les osidases, les peptidases, les sulfatases ou les phosphatases posent des problèmes de compatibilité avec les milieux de culture pour micro-organismes et en particulier pour les bactéries possédant ces activités. De plus, de tels substrats ne sont pas stables dans le temps, ce qui induit une diminution de la sensibilité vis-à-vis de l'activité enzymatique concernée avec l'allongement du temps de conservation.

Dans ce contexte, l'on connaît au travers de l'article scientifique intitulé *"*Synthèse de substrats indigogéniques. Mise en évidence de l'activité estérasique des salmonelles" : A. Agban et al., Eur. J. Med. Chem. (1990) 25, 697-699*,* des milieux de culture gélosés comprenant des substrats indigogéniques, à savoir notamment le pélargonate (C9) de bromo-5-indoxyle et un sel biliaire, à savoir le désoxycholate de sodium. De tels milieux de culture souffrent des mêmes inconvénients que ceux évoqués ci-dessous en référence au document FR-A-2697028.

La demande de brevet FR-A-2697028 divulgue un milieu de culture pour la mise en évidence de salmonelles comprenant un substrat d'estérase chromogène constitué par un ester de l'acide caprylique avec un reste indole (5-bromo-4-chloro-3-indolyl-caprylate), ainsi qu'un détergent choisi parmi les sels biliaires (désoxycholate de sodium). Ce chromogène et ce sel biliaire sont contenus dans un milieu nutritif permettant la croissance des salmonelles. Selon l'enseignement de la demande de brevet FR-A-2697028, le sel biliaire est ajouté directement au milieu sélectif dans lequel se trouve déjà inclus le substrat d'estérase. Toutefois, ce milieu de culture n'offre pas toutes les garanties souhaitables en termes de stabilité du substrat d'estérase. En outre, il s'avère que ce dernier n'est pas complètement miscible avec le milieu de culture. Cela nuit, de manière évidente, à la qualité des résultats obtenus sur le plan de la sensibilité (intensité de la coloration obtenue), de la rapidité et de la stabilité. Il convient également de noter que le milieu de culture selon FR-A-2697028 se présente sous forme de poudre. Cela oblige l'utilisateur à exécuter une opération de reconstitution préalable du milieu liquide ou gélifié. Cette contrainte résulte du manque de stabilité des substrats d'estérase mis en oeuvre.

Un autre inconvénient lié à l'emploi de sels biliaires - tels que le désoxycholate de sodium - tient au fait que ces derniers sont des matières premières d'origine animale, ce qui sous-tend une certaine variabilité en termes de qualité.

En outre, les résultats obtenus avec le milieu de culture selon FR-A-2697028 sont perfectibles en termes d'activité biologique.

Le brevet EP-B-1334206, au nom de la Demanderesse, décrit un milieu de détection/d'identification de micro-organismes (et notamment de bactéries et/ou de levures) à activité enzymatique choisie parmi les activités estérases et/ou osidases et/ou peptidases et/ou sulfatases et/ou phosphatases. Ce milieu, sous forme liquide ou gélosé, prêt à l'emploi et stable à la conservation, comprend notamment :
- au moins un substrat d'estérases, d'osidases et/ou de peptidases et/ou de sulfatases et/ou de phosphatases, chromogène ou fluorogène, et
- au moins un ester de sorbitan et d'acide(s) gras (ESAG), ou au moins un acide gras (AG) ou un mélange ESAG/AG, à titre d'agent stabilisant émulsifiant (dans un certain pourcentage en poids),
- et éventuellement au moins un Solvant (S).

Toujours selon EP-B-1334206, l'ESAG est sélectionné, de préférence, dans le groupe comprenant :
- le monolaurate de sorbitan polyoxyéthyléné comprenant 20 motifs oxyde d'éthylène (O.E), -TWEEN® 20-;
- le monopalmitate de sorbitan polyoxyéthyléné (20 O.E), -TWEEN® 40-;
- le monostéarate de sorbitan polyoxyéthyléné (20 O.E), -TWEEN® 60-;
- le tristéarate de sorbitan polyoxyéthyléné (20 O.E), -TWEEN® 65-;
- le monooléate de sorbitan polyoxyéthyléné (20 O.E), -TWEEN® 80-;
- le sesquioléate de sorbitan polyoxyéthyléné (20 O.E), -TWEEN® 83-;
- le trioléate de sorbitan polyoxyéthyléné (20 O.E), -TWEEN® 85-;
et leurs mélanges.

Les esters de sorbitan et d'acides gras (ESAG) sont des tensioactifs connus, largement utilisés dans les préparations alimentaires et pharmaceutiques. A titre illustratif, l'on peut citer l'article de DICKINSON et al. :"J Colloid interface Sci 1999 Apr 15 ; 212 (2) : 466-473*"* concernant la stabilisation d'émulsions contenant du caséinate de sodium et un monolauréate de sorbitan polyoxyéthyléné comprenant 20 motifs d'oxyde d'éthylène (TWEEN® 20). Les émulsions considérées sont des émulsions huile dans eau (30% volumique de n-tétradécane à pH 6,8).

Bien que la sensibilité de détection du milieu réactionnel selon EP-B-1334206 soit relativement satisfaisante, il existe un besoin de mettre au point un milieu de détection de micro-organismes basé sur la mise en évidence d'une activité enzymatique microbienne choisie parmi les activités estérases et/ou osidases et/ou peptidases et/ou sulfatases et/ou phosphatases de micro-organismes et offrant des performances supérieures à celles obtenues en mettant en oeuvre les milieux de l'art antérieur, et notamment celui objet du brevet EP-B-1334206. Par performances supérieures, l'on entend notamment une sensibilité de détection accrue, à savoir des intensités de coloration et/ou de fluorescence les plus élevées possibles pour les colonies des micro-organismes cibles. Ceci s'avère être tout particulièrement souhaitable en ce qui concerne la détection des salmonelles via la mise en évidence d'une activité estérase. Par ailleurs, et outre le critère de sensibilité requis, il est également important que le milieu de détection de micro-organismes susmentionné soit fiable, spécifique et reproductible.

La demande de brevet EP-A1-2107119 divulgue une composition pour la perméabilisation cellulaire, utile pour induire la pénétration de marqueurs fluorogènes à l'intérieur d'une cellule cible tout en préservant la viabilité de cette dernière, cette composition comprenant:
- une concentration finale (poids volume) d'au moins 0,01% de N-octyl-β-D-glucopyranoside (NOG);
- une concentration finale (poids volume) d'au moins 0,1% de sodium polyphosphates (HMP); et
- une concentration finale (moles/litre) d'au moins 1 mmol/L de chlorure de rubidium (RbCl) et/ou chlorure de lithium (LiCl).

En outre, la composition selon ce document peut comprendre au moins un marqueur fluorogène, lequel peut être compris directement au sein de ladite composition ou stocké séparément ou appliqué directement, lors de l'étape de détection des cellules perméabilisées par la susdite composition.

Un objectif de la présente invention est de mettre au point un milieu de détection de micro-organismes (notamment aux fins de leur caractérisation et/ou de leur dénombrement) basé sur la mise en évidence d'une activité enzymatique microbienne choisie parmi les activités estérases et/ou osidases et/ou peptidases et/ou sulfatases et/ou phosphatases de micro-organismes, qui comporte au moins un substrat d'enzyme choisi parmi les substrats d'estérases et/ou les substrats d'osidases et/ou les substrats de peptidases et/ou les substrats de sulfatases et/ou les substrats de phosphatases, chromogène et/ou fluorogène et qui soit stable à la conservation (intensité de la coloration ou de la fluorescence de révélation maintenue à un niveau maximum au moins pendant plusieurs semaines). Un autre objectif de l'invention est d'obtenir un milieu de détection de micro-organismes basé sur la mise en évidence d'une activité enzymatique microbienne choisie parmi les activités estérases et/ou osidases et/ou peptidases et/ou sulfatases et/ou phosphatases de micro-organismes, qui ne se présente pas sous forme de poudre sèche à régénérer avec un liquide pour reconstituer un milieu liquide ou gélifié, mais qui existe directement sous des formes prêtes à l'emploi.

Un autre objectif de l'invention vise à réduire les quantités de substrat(s) enzymatique(s) chromogène(s) et/ou fluorogène(s) utilisées, lequel/lesquels est/sont connu(s) de l'homme du métier pour être particulièrement onéreux.

Encore un autre objectif de l'invention consiste à mettre au point un procédé d'obtention du milieu de détection susvisé qui soit simple à mettre en oeuvre et permette notamment de caractériser les micro-organismes recherchés (notamment via leur identification et/ou la détermination de leurs propriétés de résistance potentielle à au moins un agent antimicrobien) et/ou d'effectuer leur dénombrement, sans difficulté excessive.

D'autres objectifs apparaîtront à la lecture de la présente demande.

### Exposé de l'invention

La présente invention vise à répondre au besoin susvisé et à atteindre tout ou partie des objectifs mentionnés supra.

En conséquence, un premier objet de la présente invention concerne un milieu de détection de micro-organismes, ladite détection étant basée sur la mise en évidence d'une activité enzymatique microbienne choisie parmi les activités estérases et/ou osidases et/ou peptidases et/ou sulfatases et/ou phosphatases de micro-organismes, de préférence ladite activité enzymatique microbienne étant une activité estérase, ledit milieu comprenant :
- au moins un substrat chromogène et/ou fluorogène spécifique de l'activité enzymatique recherchée, de préférence spécifique d'une activité estérase,
- au moins un composé de type alkylglycoside ou alkylthioglycoside dans lequel la partie alkyle est un radical aliphatique lineaire,
- au moins un solvant (S);
dans lequel
Selon un mode de réalisation préféré, lorsque le milieu de détection de micro-organismes selon l'invention comprend du n-octyl-β-D-glucopyranoside en tant qu'alkylglycoside, ledit milieu ne comprend pas de composé(s) compris dans le groupe constitué par les polyphosphates de sodium (HMP), le chlorure de rubidium (RbCl) et le chlorure de lithium (LiCl).

Un autre objet de la présente invention concerne un milieu de détection de micro-organismes, ladite détection étant basée sur la mise en évidence d'une activité enzymatique microbienne choisie parmi les activités estérases et/ou osidases et/ou peptidases et/ou sulfatases et/ou phosphatases de micro-organismes, de préférence ladite activité enzymatique microbienne étant une activité estérase, ledit milieu consistant essentiellement en :
- au moins un substrat chromogène et/ou fluorogène spécifique de l'activité enzymatique recherchée, de préférence spécifique d'une activité estérase,
- au moins un alkyl(thio)glycoside,
- au moins un solvant (S), et éventuellement un milieu de culture adapté pour permettre la croissance des micro-organismes recherchés (micro-organisme cibles).

L'invention a également pour objet un milieu de détection de micro-organismes, ladite détection étant basée sur la mise en évidence d'une activité enzymatique microbienne choisie parmi les activités estérases et/ou osidases et/ou peptidases et/ou sulfatases et/ou phosphatases de micro-organismes, de préférence ladite activité enzymatique microbienne étant une activité estérase, ledit milieu consistant en :
- au moins un substrat chromogène et/ou fluorogène spécifique de l'activité enzymatique recherchée, de préférence spécifique d'une activité estérase,
- au moins un alkyl(thio)glycoside,
- au moins un solvant (S),
- un milieu de culture adapté pour permettre la croissance des micro-organismes recherchés (micro-organisme cibles).

En pratique, l'homme du métier choisira le milieu de culture en fonction des micro-organismes cibles (et en particulier en fonction des bactéries cibles), selon des critères parfaitement connus et à la portée de cet homme du métier. Ce milieu de culture ne comprend pas de composé(s) compris dans le groupe constitué par les polyphosphates de sodium (HMP), le chlorure de rubidium (RbCl) et le chlorure de lithium (LiCl).

Le terme alkyl(thio)glycoside doit être compris, au sens de la présente invention, comme pouvant désigner un composé de type alkylglycoside (à savoir dans lequel la partie alkyle est reliée au résidu glucidique via une liaison éther -O-) ou de type alkylthioglycoside (à savoir dans lequel la partie alkyle est reliée au résidu glucidique via une liaison thioéther -S-), raison pour laquelle la racine « thio » figure entre parenthèses dans ce terme.

Ladite partie alkyle est généralement aliphatique, linéaire ou ramifiée, saturée ou insaturée. Par partie alkyle « aliphatique », l'on entend, au sens de la présente invention, une partie alkyle linéaire ou ramifiée ouverte (acyclique). Selon un mode de réalisation préféré, la partie alkyle du composé alkyl(thio)glycoside est un radical aliphatique linéaire (non ramifié), avantageusement saturé.

Le résidu glucidique, quant à lui, peut être un résidu d'ose (monosaccharide) ou d'oside (saccharide). La terminologie alkyl(thio)glycoside couvre donc, au sens de la présente invention, à la fois les alkyl(thio)glycosides et les alkyl(thio)polyglycosides, pouvant être utilisés seuls ou éventuellement en association avec d'autres tensioactifs, tels qu'un ou plusieurs tensioactif(s) anionique(s).

Ces alkyl(thio)glycosides sont des tensioactifs non-ioniques traditionnellement utilisés dans une large gamme d'applications industrielles et notamment en détergence ou en cosmétique.

Sans être lié par la théorie qui suit, le ou les alkyl(thio)glycoside(s) utilisé(s) aux fins de la présente invention apparaisse/apparaissent jouer le rôle d'agent(s) stabilisant(s)-émulsifiant(s). Ils peuvent être associés, selon un mode de réalisation particulier, à au moins un co-agent synergique, de préférence, au moins un tensioactif anionique, de préférence le 7-éthyl-2-méthyl-4-undécylhydrogénosulfate ou au moins l'un de ses sels et plus particulièrement ses sels de sodium (TERGITOL-4®).

Les différents procédés d'obtention des alkyl(thio)glycosides selon la présente invention sont bien connus de l'homme du métier. Ce dernier pourra donc, au choix, se les procurer dans le commerce ou les synthétiser en faisant appel à ses connaissances générales ou en se basant sur les publications existant à ce sujet.

Avantageusement, l'on utilise, en tant qu'alkyl(thio)glycoside, au moins un alkylglycoside.

Le « milieu de détection» selon l'invention doit être compris comme pouvant consister en :
- un milieu permettant de détecter/déceler la présence ou l'absence de micro-organismes, en particulier de micro-organismes recherchés (micro-organismes cibles), et/ou
- un milieu permettant de caractériser lesdits micro-organismes, à savoir notamment les identifier et/ou déterminer leurs propriétés de résistance potentielle à au moins un agent antimicrobien (agent antibiotique dans le cas de bactéries), et/ou
- un milieu permettant d'effectuer le dénombrement desdits micro-organismes ; ce dénombrement consistant à comptabiliser le nombre de colonies de micro-organismes ayant poussé sur le milieu réactionnel selon l'invention, en mettant en oeuvre des techniques de microbiologie bien connues de l'homme du métier.

L'invention concerne donc également un milieu de caractérisation (par exemple d'identification) et/ou de dénombrement de micro-organismes, ladite caractérisation et/ou ledit dénombrement étant basé(e)(s) sur la mise en évidence d'une activité enzymatique microbienne choisie parmi les activités estérases et/ou osidases et/ou peptidases et/ou sulfatases et/ou phosphatases de micro-organismes, de préférence ladite activité enzymatique microbienne étant une activité estérase, ledit milieu comprenant :
- au moins un substrat chromogène et/ou fluorogène spécifique de l'activité enzymatique recherchée, de préférence spécifique d'une activité estérase,
- au moins un alkyl(thio)glycoside, de préférence au moins un alkylglycoside, et
- au moins un solvant (S).

Selon un mode de réalisation préféré, l'activité enzymatique mise en évidence (recherchée) est une activité estérase de type hydrolase d'ester carboxylique (« carboxylic-ester hydrolase » en langue anglaise) telle qu'une activité carboxylestérase, lipase ou phospholipase.

Les micro-organismes détectés en utilisant le milieu de culture selon l'invention sont, de préférence, des bactéries et/ou des levures, avantageusement des bactéries.

Selon un mode de réalisation particulièrement préféré, le milieu réactionnel selon l'invention est utilisé pour détecter des micro-organismes (tels que des bactéries et/ou des levures) à activité estérase et, de préférence, des bactéries à activité estérase telles que des salmonelles (par exemple aux fins de leur caractérisation et/ou dénombrement). Comme cela est connu de l'homme du métier, l'activité enzymatique estérase est très répandue dans le domaine de la microbiologie. En effet, de nombreuses bactéries sont connues comme possédant une activité estérase et donc capables de cliver les substrats synthétiques (chromogéniques et/ou fluorogéniques) spécifiques d'une telle activité enzymatique. Outre les salmonelles précédemment mentionnées, l'on peut citer également les bactéries appartenant aux genres *Pseudomonas, Acinetobacter, Listeria*, etc...

La détection des micro-organismes susvisés consiste à déceler (visualiser) leur présence dans/sur un milieu de culture, via une détection visuelle ou optique de coloration(s) et/ou fluorescence(s) apparue(s) dans le milieu liquide, ou au niveau des colonies formées par ces micro-organismes sur un milieu gélosé. Dans le cas d'une détection optique, cette dernière peut être réalisée par lecture optique de tout ou partie dudit milieu, à l'aide de dispositifs tels qu'une caméra. La détection de coloration(s) et/ou fluorescence(s) par lecture optique autorise une automatisation partielle ou totale du procédé de détection correspondant.

Les substrats enzymatiques fluorogènes peuvent être de différentes natures. Tout d'abord, les substrats à base d'umbelliférone ou d'aminocoumarine et ses dérivés substitués en position 3, 4 ou 6, permettent la libération d'un composé fluorescent de couleur variant du bleu au vert sous lampe à ultraviolets (UV) (λex= 365 nm).

Ensuite, il existe des substrats à base de résorufine (et ses dérivés) résultant en la libération d'un composé fluorescent rose sous lumière naturelle (λex= 530 nm).

L'on peut également citer les substrats à base de fluorescéine (et ses dérivés) qui, après dégradation, libèrent un composé fluorescent jaune sous lumière naturelle (λex= 485 nm).

Ces substrats enzymatiques fluorogènes sont généralement peu adaptés à une utilisation dans des milieux gélosés, et sont préférentiellement utilisés en milieu liquide.

Les substrats enzymatiques chromogènes utilisables au sens de la présente invention peuvent être de différentes natures.

Premièrement, il convient de mentionner les substrats à base d'indoxyl et ses dérivés qui, en présence d'oxygène, produisent un précipité variant du bleu au rose, ainsi que les dérivés d'ALDOL™ (BIOSYNTH AG) - mentionnés dans la demande internationale WO 2010/128120 - qui produisent un précipité coloré variant du jaune au rouge y compris en l'absence d'oxygène. Ces substrats à base d'indoxyl et ses dérivés sont particulièrement préférés au sens de la présente invention du fait de leur mise en oeuvre relativement aisée et de leur bonne sensibilité de détection. Leurs applications concernent essentiellement les activités enzymatiques de type osidase, estérase et phosphatase. Bien adaptés à une utilisation sur support solide ou semi-solide (filtre, gélose, gel d'électrophorèse, etc.), ils le sont moins à une utilisation en milieu liquide (formation d'un précipité).

Deuxièmement, il existe des substrats enzymatiques à base d'hydroxyquinoline, dihydroxyflavone, dihydroxyanthraquinone, catéchol ou d'esculétine et leurs dérivés qui, en présence de sels de fer, produisent un précipité coloré. Là encore, leurs applications concernent essentiellement des activités enzymatiques de type osidase et estérase.

Troisièmement, l'on peut mentionner les substrats enzymatiques à base de nitrophénol et nitroaniline et leurs dérivés, lesquels résultent en la formation d'un composé de couleur jaune. Ils permettent de détecter des activités osidases et estérases dans le cas de substrats à base de nitrophénol et des activités peptidases dans le cas de substrats à base de nitroaniline. Cependant, dans le cas de la détection d'activités peptidases, la nitroaniline libérée est toxique pour les bactéries que l'on souhaite identifier ou caractériser, ce qui peut s'avérer préjudiciable pour des analyses en cours ou ultérieures. D'autre part, ils sont généralement peu adaptés à une utilisation sur support solide et mieux adaptés à une utilisation en milieu liquide.

Quatrièmement, il existe des substrats enzymatiques à base de naphtol et naphthylamine et ses dérivés. Dans ce cas, la réaction enzyme-substrat s'effectue en deux temps, le naphtol ou naphthylamine libéré par l'activité enzymatique subit un "azo-coupling" en présence d'un sel de diazonium qui est ajouté au moment de la révélation, conduisant à la formation d'un composé insoluble coloré. Ils permettent de détecter les activités osidases et estérases par l'intermédiaire du naphtol et les activités peptidases par l'intermédiaire de la naphthylamine. La réaction d'"azo-coupling" s'effectue dans un milieu souvent chimiquement agressif, toxique pour les bactéries et rendant l'échantillon inutilisable pour d'autres analyses, de plus les naphthylamines sont cancérigènes.

Au sens de la présente invention, les micro-organismes ayant poussé sur ou dans le milieu de détection selon l'invention sont détectés et/ou dénombrés - de visu ou par l'intermédiaire d'un dispositif optique et électronique de type caméra ou appareil photographique - via l'apparition de réactions colorées et/ou fluorescentes (selon que l'on utilise un substrat chromogène, fluorogène) ou présentant les deux caractéristiques à la fois, lesdites réactions colorées et/ou fluorescentes étant produites par l'activité enzymatique microbienne ciblée.

Concernant plus spécifiquement les substrats enzymatiques d'estérases, ces derniers comprennent, selon un mode de réalisation préféré, 2 à 16 atomes de carbone, la longueur de la chaine définissant l'application visée. Ainsi, pour détecter un maximum de germes « substrat universel », l'utilisation de substrats en C2 ou C4 est adaptée. Pour des applications plus spécifiques, comme notamment la détection des *Salmonella,* les substrats en C7-C10 sont particulièrement adaptés. Parmi les chromogènes susceptibles d'être liés à une chaine carbonée C2-C12, l'on peut citer, par exemple, les substrats à base d'indoxyl tels que 5-bromo-4-chloro-3-indoxyl, 5-bromo-6-chloro-3-indoxyl, 6-chloro-3-indoxyl, 5-bromo-3-indoxyl, 5-iodo-3-indoxyl, 6-bromo-3-indoxyl, 5,6-dibromo-3-indoxyl, les substrats à base de dihydroxyanthraquinone tel que l'alizarine ; les substrats à base d'ALDOL™ (développés par la société Biosynth AG, Rietlisstrasse 4,9422 Staad, Switzerland, mentionnés notamment au sein de la demande de brevet internationale WO 2010/128120) ; les fluorophores étant par exemple de la 4-méthylumbelliférone, et autres dérivés de 7-hydroxycoumarine.

De tels substrats enzymatiques d'estérases peuvent être, par exemple, des substrats esters chromogènes dérivés d'indoxyl et notamment le 5-bromo-4-chloro-3-indoxyl caprylate, 5-bromo-6-chloro-3-indoxyl caprylate, 5-bromo-3-indoxyl nonanoate, le 6-chloro-3-indoxyl nonanoate ou le 5-bromo-3-indoxyl décanoate. A cet égard, il convient également de mentionner les substrats d'estérases chromogènes dérivés de l'anthraquinone tels que le 2-alizarine octanoate.

En tout état de cause, ces substrats synthétiques chromogènes et/ou fluorogènes sont bien connus de l'homme du métier, lequel saura sélectionner le ou les substrat(s) enzymatique(s) à utiliser en fonction de l'activité ou des activités enzymatique(s) recherchée(s).

Selon un mode de réalisation particulièrement préféré de la présente invention, l'alkyl(thio)glycoside répond à la formule générale (I) :

R-X-(G)ₙ (I)

dans laquelle :
- R représente un radical aliphatique, linéaire ou ramifié, saturé ou insaturé, de préférence linéaire, avantageusement linéaire et saturé, comportant de 2 à 12 atomes de carbone, de préférence de 6 à 12 atomes de carbone, et préférentiellement de 8 à 12 atomes de carbone,
- X est -O- ou -S-, de préférence -O-,
- G représente un résidu glucidique,
- n est un nombre entier compris entre 1 et 10, de préférence compris entre 1 et 3, avantageusement n est le nombre entier 1 ou 2.

Une telle définition du ou des alkyl(thio)glycoside(s) selon l'invention permet d'obtenir le niveau de sensibilité de détection souhaitée, à savoir permet d'obtenir de très bonnes intensités de coloration et/ou de fluorescence à partir des substrats enzymatiques chromogènes et/ou fluorogènes utilisés. Sans être lié par la théorie, il est vraisemblable que ce/ces alkyl(thio)glycoside(s) - en particulier ceux de formule générale (I) - améliorent la détection des expressions enzymatiques ciblées, à savoir les activités estérases et/ou osidases et/ou peptidases et/ou sulfatases et/ou phosphatases microbiennes.

Le milieu de détection selon la présente invention peut comprendre un alkyl(thio)glycoside ou une pluralité d'alkyl(thio)glycosides (à savoir au moins deux alkyl(thio)glycosides), lesquels répondent, de préférence, à la formule générale (I) mentionnée supra. Lorsque le milieu de détection comprend au moins deux alkyl(thio)glycosides, ces derniers peuvent être identiques ou différents, répondant tous, de préférence, à ladite formule générale (I).

Les alkyl(thio)glycosides préférés, au sens de la présente invention, sont les alkylglycosides suivants : le n-octyl-β-D-glucopyranoside et le n-dodecyl-β-D-maltoside (ce dernier étant particulièrement préféré).

Le milieu de détection de micro-organismes selon la présente invention est susceptible d'être obtenu par mélange d'au moins un substrat chromogène et/ou fluorogène spécifique de l'activité enzymatique recherchée (de préférence spécifique d'une activité estérase) et d'au moins un alkyl(thio)glycoside tel que défini précédemment dans le solvant (S).

De manière tout à fait intéressante et notamment au regard des problématiques mentionnées dans le préambule de la présente demande, le milieu de détection de micro-organismes selon l'invention est sous forme prête à l'emploi (liquide ou gélosée) et stable à la conservation, ce qui signifie que l'intensité de coloration et/ou de fluorescence est maintenue stable au moins pendant plusieurs semaines, avantageusement pendant au moins trois semaines.

De préférence, la concentration du ou des alkyl(thio)glycoside(s) dans le milieu est comprise entre 0,5 g/L et 8 g/L, de préférence entre 0,5 g/L et 6 g/L, en fonction de l'alkyl(thio)glycoside ou des alkyl(thio)glycosides utilisé(s).

Le solvant (S) est un auxiliaire de solubilisation du substrat enzymatique chromogène et/ou fluorogène d'intérêt, en particulier en ce qui concerne un substrat enzymatique chromogène. Il complète également l'action du ou des alkyl(thio)glycoside(s) qui joue(nt) le rôle d'agent(s) stabilisant(s)-émulsifiant(s).

Selon un mode de réalisation avantageux de la présente invention, le solvant (S) est sélectionné dans le groupe comprenant :
- les alcools, de préférence le méthanol, l'éthanol, le méthoxyéthanol,
- les solvants aprotiques polaires, de préférence le diméthylformamide (DMF), le diméthylsulfoxyde (DMSO),
- les solvants aqueux, de préférence l'eau ou l'eau tamponnée,
- et leurs mélanges ;
de préférence ledit solvant (S) est le diméthylsulfoxyde (DMSO).

En pratique, les solvants préférentiellement utilisés sont le méthanol ou un solvant aprotique polaire, préférentiellement le DMF et le DMSO (de préférence le DMSO). En particulier, lorsque l'on utilise des substrats d'estérase, ces derniers sont dissous dans un solvant organique de type DMSO, auquel l'on rajoute le ou les alkyl(thio)glycoside(s) d'intérêt.

S'agissant du substrat chromogène et/ou fluorogène, ce dernier comprend une partie cible spécifique de l'enzyme à mettre en évidence, préférablement une partie cible spécifique d'une activité estérase, et une partie marqueur chromophore et/ou fluorophore, ladite partie marqueur émettant une lumière et/ou une fluorescence lorsqu'elle n'est plus associée à ladite partie cible, à savoir après clivage par ladite enzyme.

Selon un mode de réalisation préféré, le substrat enzymatique est un substrat chromogène ou fluorogène, constitué d'une partie cible de l'enzyme à détecter et d'une partie chromophore ou fluorophore, la partie cible étant sélectionnée, de préférence, dans le groupe comprenant notamment :
- les glycosides, constitués par des unités mono-, di-et/ou polysaccharides, liés en α ou β à la fonction hydroxyle de la partie fluorophore ou chromophore ;
- les acides α-aminés ou des peptides ;
- les acides organiques, tels que -O-CO(CH₂)ₙ-CH₃, avec n compris entre 0 et 20 ;
- les acides inorganiques, tels que sulfate, phosphate, pyrosulfate, pyrophosphate ou phosphodiester ;
- les Quinones/Anthraquinones et dérivés, notamment la Dihydroxyanthraquinone (Alizarine) ;
- les amino- ou hydroxy- coumarines et dérivés ;
- les fluorescéines et dérivés ;
- les indoxyls ou ALDOL™ et dérivés ;
- les aminophénols et dérivés ;
- les nitrophénols et dérivés ;
- les amino ou hydroxy-phényls et dérivés ;
- les phénoxazinones et dérivés ;
- les catéchols et dérivés ;
- les quinazolinones et dérivés (parmi lesquels ELF®97) ;
- la dihydroxyflavone et dérivés ;
- la 3-hydroxyflavone (3-HF) et dérivés ;
- l'esculétine et dérivés.

Préférablement, le substrat enzymatique est un substrat à base d'indoxyl ou d'un de ses dérivés. Dans ce cas, l'invention concerne également un milieu de détection adapté pour être utilisé en conditions d'anaérobie et/ou de microaérophilie (concentration en oxygène moléculaire dans le milieu réactionnel inférieure à la concentration atmosphérique), ledit milieu comprenant un agent favorisant la polymérisation oxydative du dérivé indoxyl, tel qu'un complexe de citrate de fer ammoniacal.

A titre d'«agents favorisant la polymérisation oxydative du dérivé indoxyl », l'on peut également citer : permanganate, Ferricyanate/Ferricyanure de potassium.

L'agent favorisant la polymérisation oxydative du dérivé indoxyl (par exemple un complexe métallique de type citrate de fer ammoniacal) est utilisé, de préférence, à une concentration comprise entre environ 0,1 et environ 2 mg/ml (de préférence de l'ordre de 0,6 mg/ml).

La concentration à laquelle le substrat enzymatique doit être utilisé dans le milieu réactionnel selon l'invention est aisément déterminable par l'homme du métier sur la base de ses connaissances générales et, le cas échéant, de tests de routine. Cette concentration doit être suffisante pour atteindre le niveau de sensibilité de détection requis mais ne doit pas être trop importante afin de ne pas risquer d'inhiber la croissance des micro-organismes.

A titre d'exemple, la concentration en substrat chromogène et/ou fluorogène est comprise entre 1 mg/L et 10 g/L, de préférence entre 5 mg/L et 6 g/L, avantageusement entre 25 mg/L et 2g/L.

Avantageusement, le milieu réactionnel selon l'invention comprend un milieu de culture adapté et connu de l'homme du métier, tel qu'un milieu décrit dans le « Handbook of culture média » (CRC Press), ledit milieu de culture étant sélectionné parmi :
- les milieux sélectifs de type MacConkey, Hektoen, milieux chromogènes sélectifs destinés à détecter sélectivement les salmonelles de type chromID® Salmonella, Columbia ANC, PALCAM, Sabouraud gentamycine-chloramphénicol, de préférence le milieu MacConkey ou un milieu chromogène sélectif destiné à détecter sélectivement les salmonelles de type chromID® Salmonella,
- les milieux non sélectifs de type Columbia +/-sang, Trypticase Soja (GTS), Gélose nutritive, Sabouraud, de préférence le milieu Columbia.

En pratique, l'homme du métier choisira le milieu de culture en fonction des micro-organismes cibles (et en particulier en fonction des bactéries cibles), selon des critères parfaitement connus et à la portée de cet homme de l'art.

Sans que cela ne soit limitatif d'une quelconque manière, il s'avère que le milieu selon l'invention est particulièrement adapté à la détection (notamment aux fins de caractérisation et/ou de dénombrement) de micro-organismes d'intérêt médical ou industriel, et notamment parmi les bactéries à Gram négatif, plus particulièrement celles du genre *Salmonella* et *Pseudomonas.*

Tel qu'indiqué au fil de la présente demande, un des objectifs principaux est de mettre au point un milieu permettant de détecter les micro-organismes (et en particulier les bactéries) possédant une activité estérase (notamment aux fins de caractérisation et/ou de dénombrement), tels que les bactéries des genres *Salmonella, Pseudomonas, Acinetobacter,* etc.

En ce qui concerne la détection des salmonelles, l'on choisira par exemple, en tant que milieu de culture, le milieu de MacConkey, le milieu Hektoen ou le milieu chromID® Salmonella.

Par ailleurs, le milieu selon l'invention peut contenir d'éventuels autres additifs comme, par exemple : un ou plusieurs autres substrat(s) enzymatique(s), par exemple chromogène(s) et/ou fluorogène(s), des peptones, un ou plusieurs facteur(s) de croissance, des hydrates de carbone, un ou plusieurs agent(s) sélectif(s), des tampons, un ou plusieurs gélifiant(s), etc.

Le milieu réactionnel selon la présente invention se présente sous une forme prête à l'emploi, de préférence sous une forme liquide ou sous forme de gel. Par forme prête à l'emploi, l'on entend une forme prête à être ensemencée en tube, flacon ou sur des boîtes de Petri.

Un des avantages du milieu réactionnel selon l'invention est qu'il est susceptible de se conserver plusieurs semaines à 4°C sous forme liquide ou de gel.

Un autre objet de la présente invention concerne un procédé d'obtention d'un milieu selon l'invention, ledit procédé comprenant les étapes suivantes :
a) préparer au moins une solution-mère d'au moins un substrat chromogène et/ou fluorogène tel que défini précédemment et d'au moins un alkyl(thio)glycoside dans le solvant (S),
b) ajouter éventuellement au moins un additif dans ledit milieu, et
c) homogénéiser l'ensemble.

Concernant l'étape a) susvisée, il convient de noter que ledit substrat et ledit au moins un alkyl(thio)glycoside peuvent être ajoutés au cours d'une même étape dans le solvant (S) ou, selon une alternative, l'on introduit, dans un premier temps, ledit substrat dans le solvant (S) puis l'on reprend ledit au moins un alkyl(thio)glycoside avec la solution précédemment obtenue (comprenant ledit substrat solubilisé par ledit solvant (S)).

Avantageusement, la préparation de la solution mère s'effectue de manière séparée en incorporant successivement le substrat enzymatique, le solvant (S) et au moins un alkyl(thio)glycoside tel que défini précédemment, éventuellement co-additivé. Les produits et quantités utilisés sont tels que définis précédemment. Après homogénéisation, la solution-mère est ajoutée au milieu de culture gélifié mis en surfusion et préalablement régénéré dans l'eau. Il peut s'agir également d'un milieu liquide non gélifié, comme par exemple un bouillon nutritif.

Après mélange du milieu de culture et de la solution mère, l'on obtient le milieu de détection liquide ou gélifié prêt à être ensemencé.

La présente invention a également pour objet l'utilisation d'un milieu selon l'invention pour la détection (notamment aux fins de caractérisation et/ou dénombrement) de micro-organismes à activité estérase et/ou osidase et/ou peptidase et/ou sulfatase et/ou phosphatase, de préférence pour la détection de micro-organismes à activité estérase tels que des bactéries du genre *Salmonella.*

Le milieu de détection selon l'invention peut être utilisé pour :
- détecter/déceler la présence ou l'absence de micro-organismes, en particulier de micro-organismes recherchés (micro-organismes cibles), et/ou
- caractériser lesdits micro-organismes, à savoir notamment les identifier et/ou déterminer leurs propriétés de résistance potentielle à au moins un agent antimicrobien (agent antibiotique dans le cas de bactéries), et/ou
- effectuer le dénombrement desdits micro-organismes.

Un autre objet de la présente invention concerne un procédé de détection de micro-organismes à activité estérase et/ou osidase et/ou peptidase et/ou sulfatase et/ou phosphatase dans un échantillon, de préférence de micro-organismes à activité estérase, ledit procédé comprenant les étapes suivantes :
i) ensemencer le milieu selon l'invention (tel que défini supra) avec un échantillon à analyser,
ii) incuber le milieu ensemencé dans des conditions appropriées (par exemple à 37°C en aérobie),
iii) détecter et interpréter les colorations et/ou fluorescences au niveau des colonies formées par les micro-organismes, lesdites colorations et/ou fluorescences révélant la réaction d'au moins un substrat chromogène et/ou fluorogène avec l'activité enzymatique microbienne qui lui est spécifique, avantageusement ladite activité enzymatique étant une activité estérase.

Ledit procédé peut être mis en oeuvre, par exemple, afin de déceler la présence ou l'absence desdits micro-organismes au sein dudit échantillon et/ou afin de les caractériser (par exemple les identifier) et/ou les dénombrer. Egalement décrit ici est l'utilisation d'au moins un alkyl(thio)glycoside pour améliorer la détection d'une activité enzymatique choisie parmi les activités estérases et/ou osidases et/ou peptidases et/ou sulfatases et/ou phosphatases de micro-organismes, de préférence ladite activité enzymatique est une activité estérase.

L'échantillon à analyser peut être de diverses origines, par exemple d'origine alimentaire, environnementale, vétérinaire ou clinique.

Parmi les échantillons d'origine alimentaire, l'on peut citer, de façon non-exhaustive, un échantillon de produits lactés (yaourts, fromages...), de viande, de poisson, d'oeuf, de fruit, de légume, d'eau, de boisson (lait, jus de fruits, soda, etc.). Bien évidemment, ces échantillons d'origine alimentaire peuvent aussi provenir de sauces ou de plats plus élaborés ou des matières premières non (ou partiellement) transformées. Un échantillon alimentaire peut enfin être issu d'une alimentation destinée aux animaux, telle que des tourteaux, des farines animales.

A titre d'exemples d'échantillons d'origine environnementale, il convient également de mentionner les prélèvements de surface, d'eau, d'air, etc.

Les échantillons biologiques d'origine clinique peuvent correspondre à des prélèvements de fluides biologiques (sang total, sérum, plasma, urine, liquide céphalo-rachidien, etc.), de selles, de prélèvements de nez, de gorge, de peau, de plaies, d'organes, de tissus ou de cellules isolées. Cette liste n'est évidemment pas exhaustive.

D'une manière générale, le terme « échantillon » se réfère à une partie ou à une quantité (plus particulièrement une petite partie ou une petite quantité) prélevée à partir d'une ou plusieurs entités aux fins d'analyse. Cet échantillon peut éventuellement avoir subi un traitement préalable, impliquant par exemple des étapes de mélange, de dilution ou encore de broyage, en particulier si l'entité de départ est à l'état solide.

L'échantillon biologique analysé est, en général, susceptible de - ou suspecté de - contenir au moins un micro-organisme cible. Dans la plupart des cas, ce dernier est un micro-organisme pathogène (tel que *Salmonella*) qu'il convient de détecter à des fins sanitaires.

Le terme « micro-organisme » a la même signification que celle généralement admise en microbiologie et comprend notamment les bactéries Gram positif ou Gram négatif, les levures, les moisissures et plus généralement, les organismes unicellulaires, invisibles à l'oeil nu, qui peuvent être manipulés et multipliés en laboratoire.

Le ou les alkyl(thio)glycoside(s) utilisé(s) aux fins de la présente invention est/sont tel/tels que défini(s) précédemment. L'on utilise un alkyl(thio)glycoside ou une pluralité d'alkyl(thio)glycosides (à savoir au moins deux alkyl(thio)glycosides), lesquels répondent, de préférence, à la formule générale (I) mentionnée supra. Lorsqu'au moins deux alkyl(thio)glycosides sont utilisés, ces derniers peuvent être identiques ou différents, répondant tous, de préférence, à ladite formule générale (I).

### Description détaillée

### Exemple 1 : milieu de détection des salmonelles - amélioration de la sensibilité de détection

### 1.1 Préparation des milieux - solubilisation des substrats enzymatiques

Une solution-mère du substrat enzymatique 5-bromo-6-chloro-3-indoxyl caprylate (magenta-C8) est réalisée dans un solvant type DMSO. Un volume de cette solution-mère permettant d'obtenir une concentration finale en substrat de 360 mg/L est ensuite ajouté dans des flacons contenant respectivement du Tween 20 (concentration finale de 6 g/L), du n-octyl-β-D-glucopyranoside (OG) aux concentrations finales de 0,7, 2 et 6 g/L, et du n-dodecyl-β-D-maltoside (DM) aux mêmes concentrations. Les différentes solutions sont agitées et les volumes introduits dans des milieux gélosés en surfusion (milieux chromID® *Salmonella*)*.* Le protocole est présenté dans le tableau 1 ci-après.

**Tableau 1**

| Ref. Milieu | T | 1 | 2 | 3 | 4 | 5 | 6 |
|---|---|---|---|---|---|---|---|
| Concentration finale en substrat dans le milieu (g/L) | 0,36 | 0,36 | 0,36 | 0,36 | 0,36 | 0,36 | 0,36 |
| Concentration finale en Tween 20 dans le milieu (g/L) | 6 | 0 | 0 | 0 | 0 | 0 | 0 |
| Concentration finale en OG dans le milieu (g/L) | 0 | 0,7 | 2 | 6 | 0 | 0 | 0 |
| Concentration finale en DM dans le milieu (g/L) | 0 | 0 | 0 | 0 | 0,7 | 2 | 6 |

La concentration finale en Tween 20 de 6g/L a été choisie car optimale dans ce contexte de solubilisation/activité microbiologique.

### 1.2 Préparation des milieux

Des micro-organismes du genre *Salmonella,* issus de la collection de la Demanderesse ont été ensemencés sur chacun des milieux susvisés selon la technique des 3 cadrans à partir de suspensions bactériennes calibrées à 0,5McF. Les souches ont été choisies pour leur expression faible à moyenne de l'activité estérase, donnant des intensités de coloration violette, faible à moyenne sur le milieu chromID® *Salmonella* commercialisé par bioMérieux (sous les références 43621 et 43629).

Les boîtes ont été incubées 24h à 37°C. Puis, les colonies formées ont été examinées visuellement après 24h d'incubation. Les intensités de coloration ont été notées. Les résultats sont consignés dans le tableau 2 ci-dessous.

**Tableau 2**

| | T | 1 | 2 | 3 | 4 | 5 | 6 |
|---|---|---|---|---|---|---|---|
| *S*. *enteritidis 0107018* | 2 | 1,5 | 3 | 4 | 4 | 4 | 3 |
| *S*. *agona 0008024* | 2 | 1,5 | 3 | 4 | 4 | 4 | 3 |
| *S*. *dublin 0008035* | 0 | 1 | 2 | 2 | 1 | 1 | 0,1 |
| *S*. *infantis 0904097* | 2 | 1,5 | 3 | 4 | 4 | 4 | 3 |
| *S*. *tennessee 0904084* | 1,5 | 1,5 | 3 | 4 | 4 | 3 | 2,5 |
| *S*. *typhimurium 0011049* | 2 | 1,5 | 3 | 4 | 4 | 4 | 3 |
| *S*. *typhimurium 0107036* | 2 | 1,5 | 2,5 | 4 | 4 | 4 | 3 |
| *S*. *tennessee 0111019* | 2 | 2 | 3 | 4 | 4 | 3 | 2,5 |
| *S*. *enteritidis 0107020* | 2 | 2 | 3 | 4 | 4 | 4 | 3 |
| *S*. *infantis 0008041* | 2 | 1,5 | 2,5 | 4 | 4 | 4 | 3 |
| *S*. *panama 0008050* | 2 | 1,5 | 3 | 4 | 4 | 4 | 3 |
| *S*. *enteritidis 0107017* | 1,5 | 1,5 | 2,5 | 3 | 4 | 4 | 3 |
| *S*. *panama 9009020* | 2,5 | 1,5 | 3,5 | 4 | 4 | 4 | 2,5 |

| | | | | | | | |
|---|---|---|---|---|---|---|---|
| *Intensités de coloration : échelle allant de 0 à 4, respectivement absence de coloration à coloration très intense;* *0 = pas de coloration* *0,1 = trace de coloration* *0,5 = coloration très pâle* *1 = coloration nette de faible intensité* *2 = coloration franche d'intensité moyenne* *3 = coloration intense* *4 = coloration très intense* *NB : Les n,5 (par exemple 1,5, 2,5, 3,5) correspondent à des intensités de coloration intermédiaires* | | | | | | | |

### 1.3 Conclusion

Les milieux 2 (OG 2 g/L), 3 (OG 6 g/L), 4 (DM 0,7 g/L), 5 (DM 2 g/L) et 6 (DM 6 g/L) permettent d'obtenir des intensités de coloration plus élevées que le milieu témoin T (Tween 20, 6 g/L) pour toutes les souches. Les milieux 1 (OG 0,7 g/L), 2 (OG 2 g/L), 3 (OG 6 g/L), 4 (DM 0,7 g/L) et 5 (DM 2 g/L) permettent de détecter toutes les salmonelles testées dont S. *dublin.* Les milieux offrant les meilleures performances sont les milieux 2 (OG 2 g/L), 3 (OG 6 g/L), 4 (DM 0,7 g/L) et 5 (DM 2 g/L).

L'OG et le DM permettent donc d'améliorer la sensibilité de détection de l'activité estérase chez *Salmonella,* vraisemblablement via une amélioration de cette expression enzymatique. En outre, ces alkylglycosides permettent également de détecter l'ensemble des souches de salmonelles testées, y compris S. *dublin.*

### Exemple 2 : détection de P. aeruginosa et A. baumannii via l'expression d'une activité estérase en C9

### 2.1 Préparation des milieux - solubilisation des substrats enzymatiques

Deux solutions-mères à 25g/L en ALDOL™ 470-nonanoate (ALDOL 470-C9, développé par la société Biosynth AG, Rietlisstrasse 4,9422 Staad, Switzerland) et en ALDOL™ 495-nonanoate (ALDOL 495-C9, également développé par la société Biosynth AG, Rietlisstrasse 4,9422 Staad, Switzerland) sont réalisées dans un solvant organique type DMSO. Ensuite, un volume correspondant à une concentration finale en substrats enzymatiques de 200 mg/L est ajouté dans des flacons contenant respectivement : Tween 20 (concentration finale dans le milieu de 6g/L), et DM (concentrations finales de 0,7, 2 et 6g/L). Les différents flacons sont vigoureusement agités puis l'intégralité du contenu est ajoutée dans les milieux gélosés en surfusion : base GTS (gélose Trypticase Soja).

La composition des différents milieux est présentée dans le tableau 3 ci-après :

**Tableau 3**

| Milieux | 1 | 2 | 3 | 4 | 5 | 6 | 7 | 8 |
|---|---|---|---|---|---|---|---|---|
| ALDOL 470-C9 g/L | 0,2 | 0,2 | 0,2 | 0,2 | 0 | 0 | 0 | 0 |
| ALDOL 495-C9 g/L | 0 | 0 | 0 | 0 | 0,2 | 0,2 | 0,2 | 0,2 |
| Tween 20 g/L | 6 | 0 | 0 | 0 | 6 | 0 | 0 | 0 |
| DM g/L | 0 | 0,7 | 2 | 6 | 0 | 0,7 | 2 | 6 |

### 2.2 Activité biologique

Des bactéries issues de la collection de la Demanderesse ont été ensemencées sur chacun des milieux susvisés selon la technique des 3 cadrans à partir de suspensions bactériennes calibrées à 0.5McF. Les boîtes ont été incubées durant 24h à 37°C et les colonies formées ont été analysées visuellement. Ainsi, les colorations et les intensités de coloration ont été notées. Les résultats sont répertoriés dans le tableau 4 ci-dessous.

**Tableau 4**

| | 1 | 2 | 3 | 4 | 5 | 6 | 7 | 8 |
|---|---|---|---|---|---|---|---|---|
| *P. aeruginosa 0001001* | 0 | 1,5 | 1,5 | 2 | 0 | 0,5 | 1,5 | 1,5 |
| *P. aeruginosa 0002019* | 0,5 | 0,5 | 0,5 | 1,5 | 0 | 0 | 0 | 0,5 |
| *P. aeruginosa 0110078* | 0,5 | 1 | 1,5 | 1,5 | 0,5 | 1 | 1 | 1,5 |
| *A. baumannii 0509060* | 0 | 1,5 | 1,5 | 2 | 0 | 1 | 2 | 2 |
| *A. baumannii 0202018* | 0 | 0,5 | 0,5 | 0,5 | 0 | 0 | 0 | 0,5 |
| *A. baumannii 0409007* | 0,5 | 2 | 2 | 2 | 0 | 1,5 | 2 | 2,5 |
| *A. baumannii 9811074* | 0 | 1 | 1,5 | 1,5 | 0 | 1 | 2 | 2,5 |
| *A. baumannii 9809057* | 0 | 0,5 | 0,5 | 0,5 | 0 | 0,5 | 1 | 1 |

| | | | | | | | | |
|---|---|---|---|---|---|---|---|---|
| Intensités de coloration : échelle allant de 0 à 4, respectivement absence de coloration à coloration très intense ; *0 = pas de coloration* *0,1 = trace de coloration* *0,5 = coloration très pâle* *1 = coloration nette de faible intensité* *2 = coloration franche d'intensité moyenne* *3= coloration intense* *4= coloration très intense* *NB : Les n,5 (par exemple 1,5, 2,5, 3,5) correspondent à des intensités de coloration intermédiaires* | | | | | | | | |

### 2.3 Conclusion

*P. aeruginosa* et *A. baumannii* sont des bactéries connues comme étant à estérase positive. Les milieux témoins 1 et 5 ne permettent pas de rendre compte de cette caractéristique. En effet, les intensités de coloration sont faibles (0,5) ou nulles pour la majorité des souches testées - avec ces 2 substrats enzymatiques à base d'ALDOL™ (et contenant du Tween 20 comme tensio-actif).

Le remplacement du Tween 20 à 6 g/L par le DM à la même concentration permet, de manière inattendue, d'améliorer l'expression de l'activité estérase. En effet, cette substitution permet de détecter toutes les souches testées, avec des intensités de coloration correctes quel que soit le substrat enzymatique testé. On note un très fort impact du DM sur l'expression de l'activité estérase lorsque le substrat est à base d'ALDOL™.

La substitution du Tween 20 par du n-dodecyl-β-D-maltoside (DM) améliore la détection de l'activité estérase des micro-organismes, vraisemblablement en raison d'une meilleure activité biologique des substrats d'estérase, ce qui se traduit par une coloration plus intense des colonies du micro-organisme cible.

L'alkylglycoside selon l'invention offre une sensibilité de détection améliorée et permet même de détecter certaines souches incolores en présence de Tween.

Par ailleurs, les intensités de coloration ou de fluorescence étant supérieures (grâce auxdits alkylglycosides), la concentration en substrat enzymatique peut ainsi être réduite, ce qui représente un avantage d'ordre économique.

### Exemple 3 : milieu de détection des salmonelles via l'utilisation d'un substrat d'estérase (lipase) à 16 atomes de carbone - amélioration de la sensibilité de détection

### 3.1 Préparation des milieux - solubilisation du substrat enzymatique

Une solution-mère du substrat enzymatique 5-bromo-4-chloro-3-indoxyl palmitate (X-C16) est réalisée dans un solvant type DMSO. Un volume de cette solution-mère permettant d'obtenir une concentration finale en substrat de 100 mg/L est ensuite ajouté dans des flacons contenant respectivement du Tween 20 (concentrations finales de 0,1%, 0,2% et 0,6% en volume), du n-octyl-β-D-glucopyranoside (OG) aux concentrations finales de 2g/L, un mélange OG (2g/L) et Tween 20 (0,1% volume), et du n-octyl- β-D-thioglucopyranoside (OTG) à la concentration finale dans le milieu de 2g/L. Les différentes solutions sont agitées et les volumes introduits dans des milieux gélosés en surfusion (milieux chromID® *Salmonella*)*.* Un récapitulatif de la composition des différents milieux est donné dans le tableau 5 ci-après.

**Tableau 5**

| Ref. Milieu | 1 | 2 | 3 | 4 | 5 |
|---|---|---|---|---|---|
| Concentration finale en X-C16 dans le milieu (g/L) | 0,1 | 0,1 | 0,1 | 0,1 | 0,1 |
| Concentration finale en Tween 20 dans le milieu (% vol.) | 0,1 | 0,2 | 0 | 0,1 | 0 |
| Concentration finale en OG dans le milieu (g/L) | 0 | 2 | 2 | 2 | 0 |
| Concentration finale en OTG dans le milieu (g/L) | 0 | 0 | 0 | 0 | 2 |

### 3.2 Ensemencement des milieux

Des micro-organismes du genre *Salmonella,* issus de la collection de la Demanderesse ont été ensemencés sur chacun des milieux susvisés selon la technique des 3 cadrans à partir de suspensions bactériennes calibrées à 0,5McF. Les souches ont été choisies pour leur différent niveau d'expression de l'activité estérase. Une souche correspondant à la souche de collection ATCC 25922 de E. *coli* sert de contrôle négatif pour l'expression d'une telle activité enzymatique.

Les boîtes ont été incubées 24h à 37°C. Puis, les colonies formées ont été examinées visuellement après 24h d'incubation. Les intensités de coloration ont été notées. Les résultats sont consignés dans le tableau 6 ci-dessous.

**Tableau 6**

| | 1 | 2 | 3 | 4 | 5 |
|---|---|---|---|---|---|
| *S. infantis 0904097* | 1 | 1,5 | 2,5 | 2,5 | 2,5 |
| *S. enteritidis 0107017* | 1 | 1 | 2,5 | 2,5 | 2 |
| *S. typhimurium 0011049* | 1 | 1 | 2,5 | 2 | 2 |
| *S*. *panama 0008050* | 1,5 | 1,5 | 2,5 | 2 | 2 |
| *S*. *panama 9009020* | 1 | 1,5 | 2,5 | 2 | 2,5 |
| *S. typhimurium 0107036* | 1,5 | 1,5 | 2,5 | 2,5 | 2 |
| *S. enteritidis 0107020* | 1 | 1 | 2,5 | 2 | 2 |
| *S. enteritidis 0107018* | 1 | 1,5 | 2,5 | 2 | 2 |
| *S. tennessee 0904084* | 1 | 1,5 | 2,5 | 2 | 2 |
| *S. infantis 0008041* | 1,5 | 1,5 | 2,5 | 2,5 | 2,5 |
| *S. dublin 0008035* | 0 | 0 | 2 | 1,5 | 0,1 |
| *S. agona 0008024* | 1 | 1 | 2,5 | 2 | 2 |
| *S. dublin 0204046* | 0 | 0 | 2,5 | 1,5 | 0 |
| *S. tennessee 0111019* | 1 | 1,5 | 2,5 | 2 | 2 |
| *E. coli 1105059* | 0 | 0 | 0 | 0 | 0 |

| | | | | | |
|---|---|---|---|---|---|
| *Intensités de coloration : échelle allant de 0 à 4, respectivement absence de coloration à coloration très intense;* *0 = pas de coloration* *0,1 = trace de coloration* *0,5 = coloration très pâle* *1 = coloration nette de faible intensité* *2 = coloration franche d'intensité moyenne* *3 = coloration intense* *4 = coloration très intense* *Remarque : les intensités notées n,5 (par exemple 1,5 et 2,5) représentent des intensités intermédiaires.* | | | | | |

### 3.3 Conclusion

Les milieux 3, 4 et 5 permettent d'obtenir des intensités de coloration plus élevées que sur le milieu témoin (milieu 1), ne comprenant ni OG, ni OTG.

On note en outre que les milieux contenant de l'OG, seul ou en mélange avec du Tween 20 (en particulier lorsque l'OG est utilisé seul comme dans le milieu 3), permettent d'obtenir les meilleures performances : sensibilité de détection de 100% sur ces milieux avec des intensités de coloration élevées pour toutes les souches dont S. *dublin.*

La souche de *E.coli,* estérase négative, n'apparait pas positive, ce qui indique que la spécificité de détection est conservée.

## Revendications

1. Milieu de détection de micro-organismes, ladite détection étant basée sur la mise en évidence d'une activité enzymatique microbienne choisie parmi les activités estérases et/ou osidases et/ou peptidases et/ou sulfatases et/ou phosphatases de micro-organismes, de préférence ladite activité enzymatique microbienne étant une activité estérase, ledit milieu comprenant :
- au moins un substrat chromogène et/ou fluorogène spécifique de l'activité enzymatique recherchée, de préférence spécifique d'une activité estérase,
- au moins un composé de type alkylglycoside ou alkylthioglycoside, dans lequel la partie alkyle est un radical aliphatique linéaire, avantageusement saturé,
- au moins un solvant (S) ;
dans lequel, lorsque ledit milieu comprend du n-octyl-β-D-glucopyranoside, ledit milieu ne comprend pas de composé(s) compris dans le groupe constitué par les polyphosphates de sodium (HMP), le chlorure de rubidium (RbCl) et le chlorure de lithium (LiCl).

2. Milieu selon la revendication 1, dans lequel ledit au moins un composé de type alkylglycoside ou alkylthioglycoside répond à la formule générale (I) :
R-X-(G)ₙ (I)
dans laquelle :
- R représente un radical aliphatique, linéaire, saturé ou insaturé, avantageusement linéaire et saturé, comportant de 2 à 12 atomes de carbone, de préférence de 6 à 12 atomes de carbone, et préférentiellement de 8 à 12 atomes de carbone,
- X est -O- ou -S-, de préférence -O-,
- G représente un résidu glucidique,
- n est un nombre entier compris entre 1 et 10, de préférence compris entre 1 et 3, avantageusement n est le nombre entier 1 ou 2.

3. Milieu selon la revendication 1 ou 2, dans lequel ledit au moins un composé de type alkylglycoside ou alkylthioglycoside est un alkylglycoside sélectionné parmi le n-octyl-β-D-glucopyranoside et le n-dodecyl-β-D-maltoside, de préférence ledit alkylglycoside consiste en le n-dodecyl-β-D-maltoside.

4. Milieu selon l'une des revendications 1 à 3, dans lequel la concentration dudit au moins un composé de type alkylglycoside ou alkylthioglycoside dans le milieu est comprise entre 0,5 g/L et 8 g/L, de préférence entre 0,5 g/L et 6 g/L.

5. Milieu selon l'une des revendications 1 à 4, dans lequel le solvant (S) est sélectionné dans le groupe comprenant :
- les alcools, de préférence le méthanol, l'éthanol, le méthoxyéthanol,
- les solvants aprotiques polaires, de préférence le diméthylformamide (DMF), le diméthylsulfoxyde (DMSO),
- les solvants aqueux, de préférence l'eau ou l'eau tamponnée,
- et leurs mélanges ;
de préférence ledit solvant (S) est le diméthylsulfoxyde (DMSO).

6. Milieu selon l'une des revendications 1 à 5, dans lequel le substrat chromogène et/ou fluorogène comprend une partie cible spécifique de l'enzyme à mettre en évidence, préférablement une partie cible spécifique d'une activité estérase, et une partie marqueur chromophore et/ou fluorophore, ladite partie marqueur émettant une lumière et/ou une fluorescence lorsqu'elle n'est plus associée à ladite partie cible, à savoir après clivage par ladite enzyme.

7. Milieu selon l'une des revendications 1 à 6, dans lequel le substrat enzymatique est un substrat à base d'indoxyl ou d'un de ses dérivés.

8. Milieu selon la revendication 7, adapté pour être utilisé en conditions d'anaérobie et/ou de microaérophilie, ledit milieu comprenant un agent favorisant la polymérisation oxydative du dérivé indoxyl, tel qu'un complexe de citrate de fer ammoniacal.

9. Milieu selon l'une des revendications 1 à 8, dans lequel la concentration en substrat chromogène et/ou fluorogène est comprise entre 1 mg/L et 10 g/L, de préférence entre 5 mg/L et 6 g/L, avantageusement entre 25 mg/L et 2g/L.

10. Milieu selon l'une des revendications 1 à 9, ledit milieu comprenant un milieu de culture adapté, ledit milieu de culture étant sélectionné parmi :
- les milieux sélectifs de type MacConkey, Hektoen, milieux chromogènes sélectifs destinés à détecter sélectivement les salmonelles de type chromID® Salmonella, Columbia ANC, PALCAM, Sabouraud gentamycine-chloramphénicol, de préférence le milieu MacConkey ou un milieu chromogène sélectif destiné à détecter sélectivement les salmonelles de type chromID® Salmonella,
- les milieux non sélectifs de type Columbia +/-sang, Trypticase Soja (GTS), Gélose nutritive, Sabouraud, de préférence le milieu Columbia.

11. Procédé d'obtention d'un milieu selon l'une des revendications 1 à 10, ledit procédé comprenant les étapes suivantes :
a) préparer au moins une solution-mère d'au moins un substrat chromogène et/ou fluorogène tel que défini dans l'une des revendications 1 à 10 et dudit au moins un composé de type alkylglycoside ou alkylthioglycoside dans le solvant (S),
b) ajouter éventuellement au moins un additif dans ledit milieu, et
c) homogénéiser l'ensemble.

12. Utilisation d'un milieu selon l'une des revendications 1 à 10 pour la détection de micro-organismes à activité estérase et/ou osidase et/ou peptidase et/ou sulfatase et/ou phosphatase, de préférence pour la détection de micro-organismes à activité estérase, tels que des bactéries ou des levures à activité estérase.

13. Utilisation selon la revendication 12, lesdits micro-organismes étant des bactéries à Gram négatif, avantageusement des bactéries du genre *Salmonella.*

14. Procédé de détection de micro-organismes à activité estérase et/ou osidase et/ou peptidase et/ou sulfatase et/ou phosphatase dans un échantillon, de préférence de micro-organismes à activité estérase, ledit procédé comprenant les étapes suivantes :
i) ensemencer le milieu selon l'une quelconque des revendications 1 à 10 avec un échantillon à analyser,
ii) incuber le milieu ensemencé dans des conditions appropriées,
iii) détecter et interpréter les colorations et/ou fluorescences au niveau des colonies formées par les micro-organismes, lesdites colorations et/ou fluorescences révélant la réaction d'au moins un substrat chromogène et/ou fluorogène avec l'activité enzymatique microbienne qui lui est spécifique, avantageusement ladite activité enzymatique étant une activité estérase.

## Patentansprüche

1. Ein Medium zum Detektieren von Mikroorganismen, wobei die Detektion auf dem Nachweisen einer mikrobiellen Enzymaktivität basiert, ausgewählt aus den Aktivitäten von Esterasen und/oder Osidasen und/oder Peptidasen und/oder Sulfatasen und/oder Phosphatasen von Mikroorganismen, wobei die mikrobielle Enzymaktivität vorzugsweise eine Esteraseaktivität ist, wobei das Medium Folgendes beinhaltet:
- mindestens ein chromogenes und/oder fluorogenes Substrat, das für die gewünschte Enzymaktivität, vorzugsweise für eine Esteraseaktivität, spezifisch ist,
- mindestens eine Verbindung der Art Alkylglykosid oder Alkylthioglykosid, wobei der Alkylteil ein linearer aliphatischer Rest ist, der vorteilhafterweise gesättigt ist,
- mindestens ein Lösungsmittel (S);
wobei das Medium, wenn das Medium n-Octyl-β-D-glukopyranosid beinhaltet, keine Verbindung(en) beinhaltet, die in der Gruppe, bestehend aus Natriumpolyphosphaten (HMP), Rubidiumchlorid (RbCl) und Lithiumchlorid (LiCl), enthalten ist/sind.

2. Medium gemäß Anspruch 1, wobei die mindestens eine Verbindung der Art Alkylglykosid oder Alkylthioglykosid der allgemeinen Formel (I) entspricht:
R-X-(G)ₙ (I)
wobei
- R einen linearen, gesättigten oder ungesättigten, vorteilhafterweise linearen und gesättigten, aliphatischen Rest mit 2 bis 12 Kohlenstoffatomen, vorzugsweise 6 bis 12 Kohlenstoffatomen und vorzugsweise 8 bis 12 Kohlenstoffatomen darstellt,
- X -O- oder -S-, vorzugsweise -O-, ist,
- G einen Kohlenhydratrest darstellt,
- n eine ganze Zahl zwischen 1 und 10, vorzugsweise zwischen 1 und 3, ist, wobei n vorteilhafterweise die ganze Zahl 1 oder 2 ist.

3. Medium gemäß Anspruch 1 oder 2, wobei die mindestens eine Verbindung der Art Alkylglykosid oder Alkylthioglykosid ein Alkylglykosid ist, das ausgewählt ist aus n-Octyl-β-D-glukopyranosid und n-Dodecyl-β-D-maltosid, wobei das Alkylglykosid vorzugsweise aus n-Dodecyl-β-D-maltosid besteht.

4. Medium gemäß einem der Ansprüche 1 bis 3, wobei die Konzentration der mindestens einen Verbindung der Art Alkylglykosid oder Alkylthioglykosid in dem Medium zwischen 0,5 g/l und 8 g/l, vorzugsweise zwischen 0,5 g/l und 6 g/l, beträgt.

5. Medium gemäß einem der Ansprüche 1 bis 4, wobei das Lösungsmittel (S) ausgewählt ist aus der Gruppe, die Folgendes beinhaltet:
- Alkohole, vorzugsweise Methanol, Ethanol, Methoxyethanol,
- polare aprotische Lösungsmittel, vorzugsweise Dimethylformamid (DMF), Dimethylsulfoxid (DMSO),
- wässrige Lösungsmittel, vorzugsweise Wasser oder gepuffertes Wasser,
- und Mischungen davon;
wobei das Lösungsmittel (S) vorzugsweise Dimethylsulfoxid (DMSO) ist.

6. Medium gemäß einem der Ansprüche 1 bis 5, wobei das chromogene und/oder fluorogene Substrat einen spezifischen Zielteil des nachzuweisenden Enzyms, vorzugsweise einen spezifischen Zielteil einer Esteraseaktivität, und einen chromophoren und/oder fluorophoren Markerteil beinhaltet, wobei der Markerteil Licht und/oder Fluoreszenz emittiert, wenn er nicht mehr mit dem Zielteil assoziiert ist, das heißt nach der Spaltung durch das Enzym.

7. Medium gemäß einem der Ansprüche 1 bis 6, wobei das Enzymsubstrat ein Substrat auf Basis von Indoxyl oder einem seiner Derivate ist.

8. Medium gemäß Anspruch 7, das zur Verwendung in anaeroben und/oder mikroaerophilen Bedingungen geeignet ist, wobei das Medium ein Mittel beinhaltet, das die oxidative Polymerisation des Indoxylderivats fördert, wie etwa ein Eisenammoniumcitratkomplex.

9. Medium gemäß einem der Ansprüche 1 bis 8, wobei die Konzentration des chromogenen und/oder fluorogenen Substrats zwischen 1 mg/l und 10 g/l, vorzugsweise zwischen 5 mg/l und 6 g/l, vorteilhafterweise zwischen 25 mg/l und 2 g/l, liegt.

10. Medium gemäß einem der Ansprüche 1 bis 9, wobei das Medium ein geeignetes Kulturmedium beinhaltet, wobei das Kulturmedium aus Folgendem ausgewählt ist:
- den selektiven Medien der Art MacConkey, Hektoen, selektive chromogene Medien zur selektiven Detektion von Salmonellen der Art chromID®-Salmonellen, Columbia CNA, PALCAM, Sabouraud-Gentamycin-Chloramphenicol, vorzugsweise einem MacConkey-Medium oder einem selektiven chromogenen Medium zur selektiven Detektion von Salmonellen der Art chromID®-Salmonellen,
- den nichtselektiven Medien der Art Columbia +/- Blut, Casein-Soja-Pepton (CASO), Nährstoff-Agar, Sabouraud, vorzugsweise dem Columbia-Medium.

11. Ein Verfahren zum Erhalten eines Mediums gemäß einem der Ansprüche 1 bis 10, wobei das Verfahren die folgenden Schritte beinhaltet:
a) Zubereiten mindestens einer Stammlösung aus mindestens einem chromogenen und/oder fluorogenen Substrat gemäß einem der Ansprüche 1 bis 10 und der mindestens einen Verbindung der Art Alkylglykosid oder Alkylthioglykosid in dem Lösungsmittel (S),
b) optionales Zugeben von mindestens einem Additiv zu dem Medium und
c) Homogenisieren des Ganzen.

12. Eine Verwendung eines Mediums gemäß einem der Ansprüche 1 bis 10 zur Detektion von Mikroorganismen mit einer Aktivität von Esterase und/oder Osidase und/oder Peptidase und/oder Sulfatase und/oder Phosphatase, vorzugsweise zur Detektion von Mikroorganismen mit Esteraseaktivität, wie etwa Bakterien oder Hefen mit Esteraseaktivität.

13. Verwendung gemäß Anspruch 12, wobei die Mikroorganismen gramnegative Bakterien sind, vorteilhafterweise Bakterien der Gattung *Salmonella.*

14. Ein Mikroorganismusdetektionsverfahren mit einer Aktivität von Esterase und/oder Osidase und/oder Peptidase und/oder Sulfatase und/oder Phosphatase in einer Probe, vorzugsweise Mikroorganismen mit Esteraseaktivität, wobei das Verfahren die folgenden Schritte beinhaltet:
i) Beimpfen des Mediums gemäß einem der Ansprüche 1 bis 10 mit einer zu analysierenden Probe,
ii) Inkubieren des beimpften Mediums unter geeigneten Bedingungen,
iii) Detektieren und Interpretieren der Färbungen und/oder Fluoreszenzen an den von den Mikroorganismen gebildeten Kolonien, wobei die Färbungen und/oder Fluoreszenzen die Reaktion von mindestens einem chromogenen und/oder fluorogenen Substrat mit der mikrobiellen Enzymaktivität, für die sie spezifisch ist, zeigen, wobei die Enzymaktivität vorteilhafterweise eine Esteraseaktivität ist.

## Claims

1. A microorganism detection medium, said detection being based on showing the presence of a microbial enzyme activity chosen from esterase and/or osidase and/or peptidase and/or sulfatase and/or phosphatase activities of microorganisms, preferably said microbial enzyme activity being an esterase activity, said medium comprising:
- at least one chromogenic and/or fluorogenic substrate specific to the enzyme activity sought, preferably specific to an esterase activity,
- at least one compound of the alkylglycoside or alkylthioglycoside type, wherein the alkyl part is a linear aliphatic radical which is advantageously saturated,
- at least one solvent (S).
wherein, when said medium comprises n-octyl-β-D-glucopyranoside, said medium does not comprise compound(s) comprised in the group constituted by sodium polyphosphates (HMP), rubidium chloride (RbCl) and lithium chloride (LiCl).

2. The medium according to claim 1, wherein said at least one compound of the alkylglycoside or alkylthioglycoside type corresponds to the general formula (I):
R-X-(G)ₙ (I)
wherein:
- R represents an aliphatic, linear, saturated or unsaturated radical, advantageously linear and saturated, containing 2 to 12 carbon atoms, preferably 6 to 12 carbon atoms, and preferably 8 to 12 carbon atoms,
- X is -O- or -S-, preferably -O-,
- G represents a glucidic residue,
- n is an integer between 1 and 10, preferably between 1 and 3, advantageously n is the integer 1 or 2.

3. The medium according to claim 1 or 2, wherein said at least one compound of the alkylglycoside or alkylthioglycoside type is an alkylglycoside selected from n-octyl-β-D-glucopyranoside and n-dodecyl-β-D-maltoside, preferably said alkylglycoside consists of n-dodecyl-β-D-maltoside.

4. The medium according to one of claims 1 to 3, wherein the concentration of said at least one compound of the alkylglycoside or alkylthioglycoside type in the medium is between 0.5 g/L and 8 g/L, preferably between 0.5 g/L and 6 g/L.

5. The medium according to one of claims 1 to 4, wherein the solvent (S) is selected from the group comprising:
- alcohols, preferably methanol, ethanol, methoxyethanol,
- polar aprotic solvents, preferably dimethylformamide (DMF), dimethylsulfoxide (DMSO),
- aqueous solvents, preferably water or buffered water,
- and mixtures thereof;
preferably said solvent (S) is dimethylsulfoxide (DMSO).

6. The medium according to one of claims 1 to 5, wherein the chromogenic and/or fluorogenic substrate comprises a target part specific to the enzyme to be shown, preferably a target part specific to an esterase activity, and a chromophoric and/or fluorophoric marker part, said marker part emitting a light and/or a fluorescence when it is no longer associated with said target part, namely after cleavage by said enzyme.

7. The medium according to one of claims 1 to 6, wherein the enzyme substrate is a substrate based on indoxyl or one of its derivatives.

8. The medium according to claim 7, suitable for use in anaerobic and/or microaerophilic conditions, said medium comprising an agent which promotes the oxidative polymerisation of the indoxyl derivative, such as a ammonium ferric citrate complex.

9. The medium according to one of claims 1 to 8, wherein the concentration of chromogenic and/or fluorogenic substrate is between 1 mg/L and 10 g/L, preferably between 5 mg/L and 6 g/L, advantageously between 25 mg/L and 2 g/L.

10. The medium according to one of claims 1 to 9, said medium comprising a suitable culture medium, preferably said culture medium being selected from:
- the selective media of the MacConkey, Hektoen types, selective chromogenic media intended for selectively detecting salmonellae of the chromID® Salmonella, Columbia CNA, PALCAM, Sabouraud gentamycin-chloramphenicol types, preferably the MacConkey medium or a selective chromogenic medium intended for selectively detecting salmonellae of the chromID® Salmonella type,
- the non-selective media of the Columbia +/- blood, Trypticase Soy (TSA), nutrient agar, Sabouraud types, preferably the Columbia medium.

11. A method of obtaining a medium according to one of claims 1 to 10, said method comprising the following steps:
a) preparing at least one stock solution of at least one chromogenic and/or fluorogenic substrate such as defined in one of claims 1 to 10 and of said at least one compound of the alkylglycoside or alkylthioglycoside type in the solvent (S),
b) possibly adding at least one additive into said medium, and
c) homogenising the whole.

12. Use of a medium according to one of claims 1 to 10 for the detection of microorganisms with esterase and/or osidase and/or peptidase and/or sulfatase and/or phosphatase activity, preferably for the detection of microorganisms with esterase activity, such as bacteria or yeasts with esterase activity.

13. The use according to claim 12, said microorganisms being Gram-negative bacteria, advantageously bacteria of the genus *Salmonella.*

14. A method of detecting microorganisms with esterase and/or osidase and/or peptidase and/or sulfatase and/or phosphatase activity in a sample, preferably microorganisms with esterase activity, said method comprising the following steps:
i) seeding the medium according to any one of claims 1 to 10 with a sample to be analysed,
ii) incubating the seeded medium in appropriate conditions,
iii) detecting and interpreting the colourings and/or fluorescences at the colonies formed by the microorganisms, said colorations and/or fluorescences revealing the reaction of at least one chromogenic and/or fluorogenic substrate with the microbial enzyme activity which is specific to it, advantageously said enzyme activity being an esterase activity.
